# EUROPEAN PATENT SPECIFICATION

(11) **EP 4 518 761 B1**
(45) Date of publication and mention of the grant of the patent: **26.11.2025**
(21) Application number: 23724021.3
(22) Date of filing: 20.04.2023
(51) Int. Cl.: A61B 5/367, A61B 18/14

(54) **DETECTING POTENTIAL SLOW-CONDUCTION CARDIAC TISSUE AREAS IN STABLE ARRHYTHMIAS**
ERKENNUNG POTENTIELLER LANGSAM LEITENDER HERZGEWEBEBEREICHE BEI STABILEN ARRHYTHMIEN
DÉTECTION DE ZONES DE TISSU CARDIAQUE À CONDUCTION DE POTENTIEL LENTE DANS DES ARYTHMIES STABLES

(30) Priority: 04.05.2022 US 202217736292
(43) Date of publication of application: 12.03.2025
(73) Proprietor: Biosense Webster (Israel) Ltd., 2066717 Yokneam (IL)
(72) Inventor: SEGEV, Meytal, 2066717 Yokneam (IL); MASSARWA, Fady, 2066717 Yokneam (IL); ALTMAN, Sigal, 2066717 Yokneam (IL)
(74) Representative: Carpmaels & Ransford LLP
(86) International application number: PCT/IB2023/054036
(87) International publication number: WO 2023/214234

(56) References cited:
- US-A1- 2017 202 521
- US-A1- 2019 328 258
- US-A1- 2020 146 579

## Description

### FIELD OF THE DISCLOSURE

This disclosure relates generally to analysis of electrophysiological (EP) signals, and specifically to a method for detecting slow conduction in stable arrhythmias, such as an atrial flutter.

### BACKGROUND OF THE DISCLOSURE

Electrophysiological (EP) mapping of cardiac regions to identify cardiac tissue regions of slow conductions was previously proposed in the patent literature. For example, International Patent Application Publication WO 2020/214439 describes an electroanatomical mapping system that can map electrical activation of tissue, and in particular create a slow-conduction map, using a plurality of electrophysiology data points, each including local activation timing information, by computing a slow-conduction metric for each point using the local activation timing information. The slow-conduction metric can be used to classify points as no conduction points, slow-conduction points, and normal conduction points, and the results can be graphically expressed, including as an animated representation of an activation wavefront propagating along a three-dimensional anatomical surface model.

US 2017/202521 A1 discloses a method of determining target heart ablation regions which includes detecting electro-cardiogram (ECG) signals each indicating an electrical activity of a heart over time and determining, for each of the ECG signals, local activation times (LATs) each indicating a time of electrical activation for an area the heart. The method also includes generating, based on the LATs, electrical activity mapping information for displaying one or more maps representing the electrical activity of the heart and alternatively, or in addition to the electrical activity mapping information, generating spatio-temporal mapping information for one or more maps representing a spatio-temporal manifestation of the conditions indicative of cardiac arrhythmia, such as atrial fibrillation (AF). The method further includes determining a region of interest (ROI) of the heart by identifying the ROI as a region exhibiting conditions indicative of cardiac arrhythmia based on the mapping information.

US 2020/146579 A1 discloses a method including receiving an input mesh representation of a cardiac chamber, a set of measured locations on a wall tissue of the cardiac chamber, and a respective set of local activation times (LATs) measured at the locations. The input mesh is re-meshed into a regular mesh including regularized polygons. The set of measured locations and respective LATs is data fitted to the regularized polygons. Respective LAT values, and respective probabilities that the wall tissue includes scar tissue, are iteratively calculated for the regularized polygons, so as to obtain an electrophysiological (EP) activation wave over the regular mesh that indicates scar tissue. An electroanatomical map overlaid on the regular mesh, the map including the EP activation wave and the scar tissue, is presented.

US 2019/328258 A1 discloses aystems and methods for quantifying cardiac electrophysiologic signals. An electronic processor receives a unipolar electrogram signal from each of a plurality of electrodes positioned at different locations of a heart. The electronic processor then calculates or measures a bipolar electrogram signal based on a difference between the unipolar electrogram signal for a first electrode and the unipolar electrogram signal for a second electrode. A local activation time (LAT) difference between a location of the first electrode and a local of the second electrode is then determined based on a voltage amplitude of the bipolar electrogram signal. The LAT difference is indicative of an amount of time between a local activation of a propagating wavefront at the location of the first electrode and a local activation of the propagating wavefront at the location of the second electrode.

### BRIEF DESCRIPTION OF THE DRAWINGS

The present disclosure will be more fully understood from the following detailed description of the examples thereof, taken together with the drawings, in which:
Fig. 1 is a schematic, pictorial illustration of a catheter-based electrophysiological (EP) mapping system, according to an example of the present disclosure;
Figs. 2A-2C are a schematic, pictorial illustrations of algorithm steps for the detection of slow-conduction cardiac tissue areas in stable arrhythmias, according to an example of the present disclosure;
Fig. 3 is a flow chart that schematically illustrates a method and algorithm for the detection of slow-conduction cardiac tissue areas in stable arrhythmias, according to an example of the present disclosure; and
Fig. 4 is a volume rendering of an EP map of a left atrium showing tissue locations of potential slow conduction that may cause a stable arrhythmia, according to an example of the present disclosure.

### DETAILED DESCRIPTION OF EXAMPLES

### OVERVIEW

A stable arrhythmia, such as an atrial flutter (AFL) or a stable ventricular tachycardia (VT), is often defined as a sustained arrhythmia not associated with significant hemodynamic compromise. However, a stable arrythmia can readily deteriorate into life-threatening conditions, and therefore, once diagnosed, it requires prompt treatment, including an option to ablate arrhythmogenic tissue locations that cause the stable arrhythmia.

In order to characterize a stable arrhythmia of a patient, a catheter-based electrophysiological (EP) mapping system may be used to generate an EP map of least part of the heart of the patient, such as an EP map of a cardiac chamber. In a typical catheter-based EP mapping procedure, a distal end of a catheter, which comprises one or more sensing electrodes, is inserted into the cardiac chamber to sense EP signals, such as bipolar electrograms (EGM). As a physician operating the system moves the distal end inside the cardiac chamber, the EP-mapping system acquires EP signals at various locations on the inner surface of the cardiac chamber, as well as the respective positions of the distal end. Based on these acquired signals, a processor of the mapping system generates the required EP map comprising local EP tissue characteristics (e.g., local cycle time between activations) overlaid on an anatomical map of the cardiac chamber.

When mapping a stable arrhythmia (e.g., an AFL), a physician looks for particular areas on a surface of cardiac tissue having aberrant EP characteristics, e.g., one or more tissue isthmuses, which are EP-conducting zones between adjacent scars or between natural structural barriers and scars. Such zones may be characterized by EP signals having slow conduction that can facilitate stable arrhythmias. The physician may therefore look on the EP map for slow-conduction zones and ablate one or more of these target zones to terminate an arrythmia. This may be used mostly in AFL but also potentially in VT cases.

However, slow-conduction areas are characterized by complex phenomenology, such as scar/line of blocked areas, synchronized wave direction, instability of local activation time (LAT), and low-amplitude bipolar signals, among others, which makes identification of most relevant slow-conduction areas difficult.

Present systems using current algorithms for identifying and characterizing a stable arrhythmia do not provide good pointers to the most likely areas for a physician to ablate, so the physician typically has to perform time-consuming checking of candidate tissue locations before ablation. In particular, current workflows to detect slow-conduction areas in AFL give no clear guidance for the most relevant features or workflow that should be used to enhance detection of those areas. Today, the physician needs to invest time to activate, configure and analyze a number of system features, and, in addition, manually tag a significant number of, for example, complex fractionated signals, as well as mark potential ablation sites.

Examples of the present disclosure that are described herein provide a method and system to provide an integrated automatic analysis that enables the user to easily detect potential slow-conduction areas. The indicated slow-conduction areas, detected by the algorithm using slow-conduction criteria comprising three steps described below, are marked (e.g., overlaid) on an EP map.

The disclosed algorithm relies on the observation that AFL and some other stable arrhythmias are caused by the occurrence of aberrant activation wave reentries due to the EP activation wave traveling in closed trajectories within the heart, rather than moving from one end of the heart to the other and then terminating. As the EP activation wave continuously propagates at some cycle length (e.g., a time between consecutive peaks in the ECG), wave propagation in a cardiac chamber can span the entire reentry cycle, where a "late" wavefront in the cycle meets the "early" wavefront of the next cycle. As a result, regions might be assigned incorrect local activation times (LATs) that are either very short or very long (a phenomenon called hereinafter "Early Meet Late," or EML).

Typically, EML areas are bound by a curve of blocked conduction, such as curves described in U.S. Patent No. 9,649,046, which is assigned to the assignee of the present application. EP map visualization is available to highlight such potential areas of blocked conduction, the potential areas of conduction block called hereinafter "EML areas," and the encircling curves of blocked conduction called "EML boundaries." An example of such a visualization of areas of blocked conduction (e.g., EML areas) in complex arrhythmias is disclosed in U.S. Patent No. 10,136,828, which is assigned to the assignee of the present application.

In some examples of the disclosed technique, a processor receives an EP map of a cardiac surface, the EP map comprising data points comprising positions on the surface and respective local activation times (LAT), as well as areas designated by early meets late (EML) LAT occurrences. The processor identifies a set of shortest paths between different EML areas where such paths may cross isthmus zones. The processor does not consider exceedingly long paths, i.e., paths between remotely located EML areas, since they are unlikely to represent valid paths across isthmuses.

Using an LAT histogram of the surface, the processor selects one or more ranges (e.g., bins of a histogram) of LAT values with the lowest prevalence of such data points. The processor generates complex fractionated tags for the positions on the EP map having LAT values within these one or more ranges of LAT values with lowest prevalence. An algorithm that places complex tags at tissue locations according to magnitude of fractionations (indicative of stable arrhythmia) is described in U.S. Patent Application No. 17/548,558, titled, "Detection of Fractionated Signals in stable arrhythmias," filed December 12, 2021. The processor removes short paths along areas with sparse complex tags within them from consideration by selecting a subset of the set's shortest paths between the EML areas, based on density of complex fractionated tags along paths.

The EP map further includes activation wave velocities. The processor further filters the subset of the shortest paths between the EML areas by selecting paths whose velocity vectors in the isthmus areas propagating in a general direction along the isthmus, and not propagating between EML areas. The propagation along an isthmus is likely to be a source of reentrant arrhythmia, whereas propagation across an isthmus is likely a result of collision of EP wavefronts therein, which is clinically irrelevant.

Finally, the processor presents the selected subset of paths as candidate slow EP conduction gaps for ablation aimed at eliminating an arrhythmia (such as AFL).

### SYSTEM DESCRIPTION

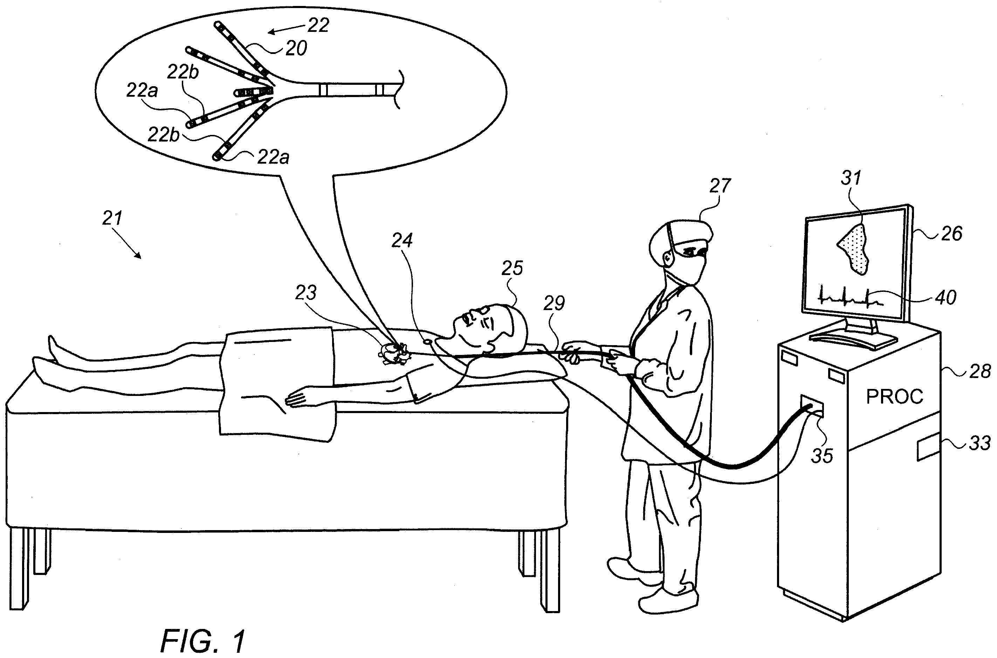

Fig. 1 is a schematic, pictorial illustration of a catheter-based electrophysiological (EP) mapping system 21, according to an example of the present disclosure. Fig. 1 depicts a physician 27 using an electro-anatomical mapping catheter 29 to perform an electro-anatomical mapping of a heart 23 of a patient 25. Mapping catheter 29 comprises, at its distal end, one or more arms 20, each of which is coupled to a bipolar electrode 22 comprising adjacent electrodes 22a and 22b. In some examples, the distal end of the catheter includes a magnetic sensor (not shown) that allows to magnetically track the locations of electrodes 22 or to calibrate the aforementioned the electrical tracking signals to improve an accuracy of an electrical location tracking method.

During the mapping procedure, the locations of electrodes 22 are tracked while they are inside heart 23 of the patient. For that purpose, electrical signals are passed between electrodes 22 and external electrodes 24. For example, three external electrodes 24 may be coupled to the patient's chest, and another three external electrodes may be coupled to the patient's back. For ease of illustration, only one external electrode is shown in Fig. 1.

Based on the signals, and given the known positions of electrodes 24 on the patient's body, processor 28 calculates an estimated location of each electrode 22 within the patient's heart. Respective electrophysiological data, such as bipolar electrogram traces, are additionally acquired from heart 23 tissue by using electrodes 22. The processor may thus associate any given signal received from electrodes 22, such as a bipolar EP signal, with the location at which the signal was acquired. The processor 28 receives the resulting signals via an electrical interface 35, and uses information contained in these signals, and stored in a memory 33, to construct an electrophysiological map 31 (that will also be stored in memory 33 for upload by processor 28) and EGM or ECG traces 40, and to present these on a display 26. One tracking system and method capable of producing map 31 is the Advanced Current Location (ACL) system, implemented in various medical applications, for example, in the CARTO^{™} system, produced by Biosense-Webster Inc., which is described in detail in U.S. Patent No. 8,456,182.

A magnetic tracking system (not shown) and method capable of producing map 31 or improving the accuracy of the ACL method is described in U.S. Patent Nos. 5,391,199, 6,690,963, 6,484,118, 6,239,724, 6,618,612 and 6,332,089, in PCT Patent Publication WO 96/05768, and in U.S. Patent Application Publication Nos. 2002/0065455 A1, 2003/0120150 A1 and 2004/0068178 A1.

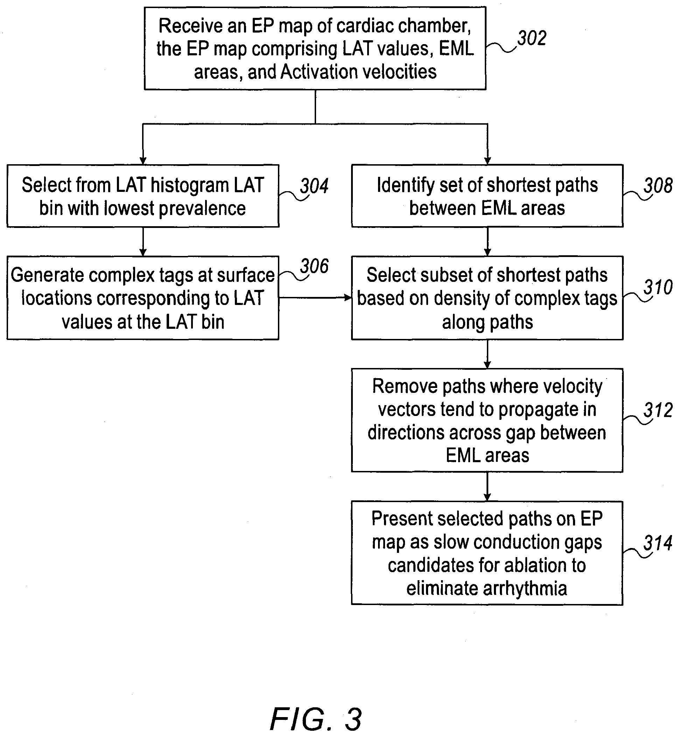

Processor 28 typically comprises a general-purpose computer with software programmed to carry out the functions described herein. The software may be downloaded to the computer in electronic form, over a network, for example, or it may, alternatively or additionally, be provided and/or stored on non-transitory tangible media, such as magnetic, optical, or electronic memory. In particular, processor 28 runs a dedicated algorithm as disclosed herein, including in Fig. 3, that enables processor 28 to perform the disclosed steps, as further described below.

The example illustration shown in Fig. 1 is chosen purely for the sake of conceptual clarity. Other types of electrophysiological sensing catheter geometries, such as the Lasso^{®} Catheter (produced by Biosense-Webster Inc., Irvine, California) may be employed. Additionally, contact sensors may be fitted at the distal end of mapping catheter 29 to transmit data indicative of the physical quality of electrode contact with tissue. In an example, measurements of one or more electrodes 22 may be discarded if their physical contact quality is indicated as poor, and the measurements of other electrodes may be regarded as valid if their contact quality is indicated as sufficient.

### DETECTION OF POTENTIAL SLOW-CONDUCTION CARDIAC TISSUE AREAS IN STABLE ARRHYTHMIAS

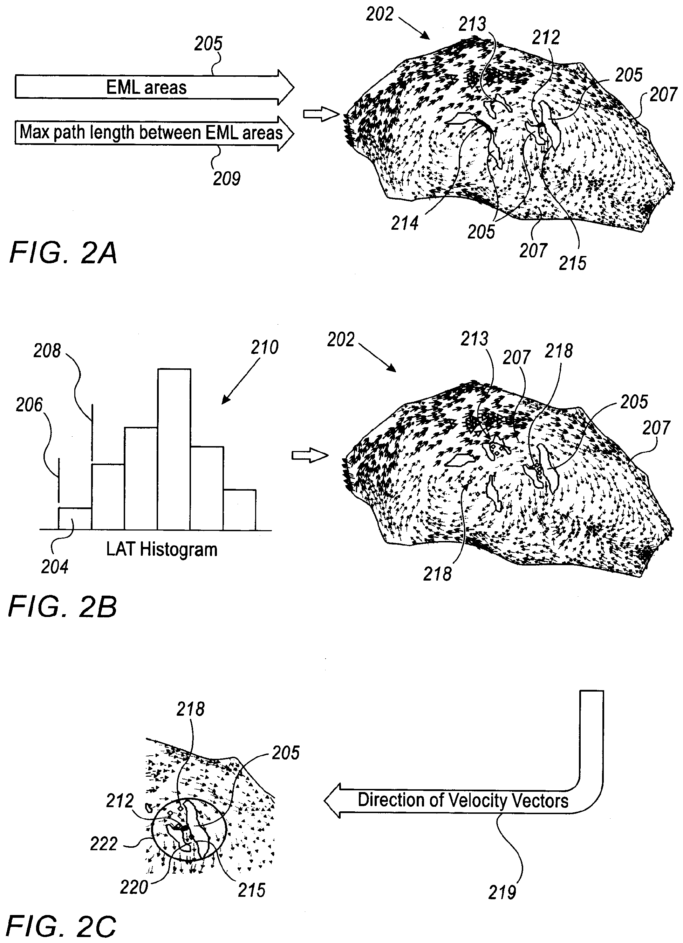

Figs. 2A-2C are a schematic, pictorial illustrations of algorithm steps for the detection of slow-conduction cardiac tissue areas in stable arrhythmias, according to an example of the present disclosure.

Figs. 2A-2C are a high-level visual description of the algorithm steps briefly described in the text above, and which are described in detail in Fig. 3.

Fig. 2A shows a set of shortest paths (212, 213, 214) identified on an EP map 202 by a processor using the disclosed algorithm. The paths are drawn over candidate slow-conduction gaps between EML areas 205. The inputs of this step comprise the EML areas 205, each defined by a curve around an area of blocked conduction, called herein an EML boundary 215, and by a predefined limit 209 on maximal path length considered (also called below "MAX_GAP_LENGTH") to remove irrelevant (e.g., too long) paths, i.e., paths between remotely located EML areas, as described above and detailed in Fig. 3.

Fig. 2B shows that paths 214 are removed from consideration using LAT histogram 210, of which a lowest prevalence bin 204 is only considered (LAT bin 204 includes data ponits in EP map 202 within LAT range [minLAT 206, maxLAT 208]). As seen on EP map 202, only complex tags 218 that are associated with LAT bin 204, are considered (i.e., filtered), which leaves for consideration only paths based on density of the relevant complex tags 218 along each path of the group of paths (212, 213).

Finally, Fig. 2C shows a selected path 212, which is left on EP map 202 and indicated (222) to a user as a path over possible slow-conduction gap 220. Path 212 is left after filtering out paths, such as filtering out (219)path 213, aside which activation wave velocities 207 show propagation largely across the gap, rather than being more perpendicular or with an angle relative to path 212, since only the latter is indicative of possible reentrant activation via a gap (e.g.,, isthmus) 220 between close EML areas 205. In other words, Fig. 2C shows path selection based on filtering out paths for which the velocity vectors are parallel to a tangent at the path, up to a given angular tolerance over the degree of parallelism. On the other hand, velocity vectors that are largely orthogonal to the path (i.e., parallel to a tangent of the EML boundary) at the EML boundary 215, where the path meets the EML area, indicate reentrant propagation that may cause an arrhythmia.

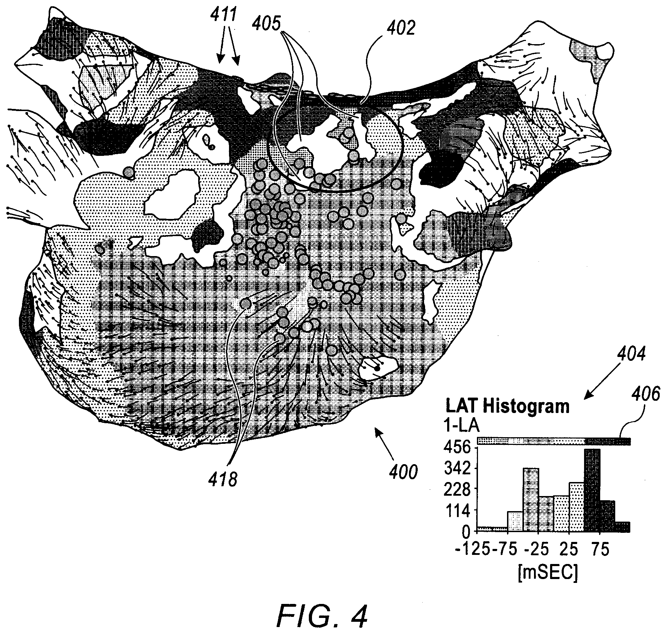

Fig. 3 is a flow chart that schematically illustrates a method and algorithm for the detection of slow-conduction cardiac tissue areas 220 in stable arrhythmias, according to an example of the present disclosure. The algorithm, according to the presented example, carries out a process that begins at an EP map receiving step 302, with a processor, such as processor 28, receiving an EP map, such as map 202, comprising (i) surface locations and respective LAT values at the locations, (ii) activation wave velocity vectors (e.g., vectors 207), and EML areas (e.g., areas 205). The surface location and LAT values are not shown explicitly in the map, rather, as shown by the EP map of Fig. 4, such EP maps are typically color coded with a continuous LAT scale interpolating between the discrete LAT values measured.

Next, the process runs steps 304 and 306 in parallel to step 308, to generate both necessary inputs for step 310.

At an LAT bin selection step 304, the processor selects one or more LAT bins of a LAT histogram of the LAT values of step 302. The processor may select a lowest prevalence LAT bin, such as bin 204 of Fig. 2B, or, for example, select both lowest and second-lowest prevalence LAT bins. Step 304 may involve calculating the LAT histogram, if such is not already available.

Next, at a complex tags generation step 306, processor 28 applies the aforementioned complex tags algorithm of U.S. Patent Application No. 17/548,558 with the [minLAT, maxLAT] bin 204 as the "time frame within WOI" to identify locations of such a subset of tags on the map surface.

In parallel, at paths identification step 308, processor 28 finds virtual shortest paths (212, 213, 214) between all EML areas 205. As a preparatory step, processor 28 utilizes an existing algorithm to find all EML areas 205, if such are not already given on EP map 202. Step 308 further excludes paths with lengths exceeding a predefined maximal path length, "MAX_GAP_LENGTH," to remove irrelevant (e.g., too long) paths which are therefore not paths over isthmuses.

At a path subset selection step 310, the processor further filters out irrelevant paths, based on a low density of tags along a path (e.g., within an area including the path).

At a final path selection step 312, processor 28 maintains only paths that meet a last step in the slow-conduction criteria, by maintaining paths for which velocity vectors along the EML boundary are consistent in direction and are parallel to the EML boundary tangent direction up to a predefined parallelism tolerance.

Finally, at paths presentation step 314, processor 28 presents the selected subset of paths as candidate slow-conduction gaps for ablation aimed at eliminating the cardiac arrhythmia.

The flow chart shown in Fig. 3 is chosen purely for the sake of conceptual clarity. Other possible steps are omitted from the disclosure herein purposely in order to provide a more simplified flow chart.

Fig. 4 is a volume rendering 400 of an EP map of a left atrium showing tissue locations of potential slow conduction that may cause a stable arrhythmia, according to an example of the present disclosure. As seen, the processor indicates (402) a region of isthmuses between EML areas 405, that are identified as crossed by paths satisfying the above described three elements of slow-conduction criteria:
1. High density of complex tags 418 in path area
2. Path length < MAX_GAP_LENGTH
3. Velocity vectors along the paths consistent in direction and not parallel to the paths' tangent direction

As seen, other possible isthmuses causing arrhythmia, such as isthmuses at regions 411, are automatically ruled out by the disclosed technique.

Fig. 4 further shows an LAT histogram 404, which is used in filtering designed to maintain only relevant complex tags 418, as described above. Further shown is a continuous LAT scale 406 of EP map 400 and histogram 404, which the physician may use to verify locations of late activation times which are indicative of an arrythmia, such as those found inside the region emphasized (402) by the disclosed technique, to consider ablation therein.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. The scope of the present invention is defined by the appended claims.

## Claims

1. A method for identifying candidate locations for ablation, the method comprising:
receiving an electrophysiological (EP) map (202) comprising an anatomical surface of a cardiac chamber overlaid with (i) activation wave velocity vectors (207), and (ii) data points comprising positions on the anatomical surface and respective local activation times (LAT),
**characterized in that** the anatomical surface of the cardiac chamber is further overlaid with (iii) areas (205) designated by early meet late (EML) LAT range; wherein the method further comprises:
identifying a set of shortest paths (212, 213, 214) on the anatomical surface between different EML areas;
selecting one or more ranges (204) of LAT values that are **characterized by** lowest prevalence over the data points of the EP map;
generating complex tags (218) for the positions having the LAT values within the one or more ranges of LAT values having the lowest prevalence;
selecting a subset of the shortest paths (212) based on (i) density of the complex tags along the shortest paths and (ii) directions of the activation wave velocity vectors relative to each of the shortest paths; and
presenting the selected subset of the shortest paths as candidate slow-conduction areas for ablation.

2. The method according to claim 1, wherein selecting the one or more ranges of the LAT values comprises selecting one or more LAT bins of a LAT histogram.

3. The method according to claim 1, wherein selecting the subset of the shortest paths comprises filtering out a path longer than a predefined path length.

4. The method according to claim 1, wherein receiving the EP map with the areas designated by early meet late (EML) LAT range comprises receiving areas bounded by a curve of blocked conduction (215).

5. The method according to claim 1, wherein selecting the shortest paths based on the direction of the activation wave velocity vectors comprises filtering out a path for which the activation wave velocity vectors are parallel to a tangent to the path, up to a given angular tolerance.

6. The method according to claim 1, wherein generating the complex tags comprises annotating electrograms at fractioned regions, and extracting LAT values using the annotated electrograms.

7. The method according to claim 1, wherein the cardiac chamber is an atrium exhibiting an atrial flutter.

8. The method according to claim 1, wherein the cardiac chamber is a ventricle exhibiting ventricular tachycardia.

9. A system for identifying candidate locations for ablation, the system comprising:
an interface (35) configured receive an electrophysiological (EP) map comprising an anatomical surface of a cardiac chamber overlaid with (i) activation wave velocity vectors (207), and (ii) data points comprising positions on the anatomical surface and respective local activation times (LAT),
**characterized in that** the anatomical surface of the cardiac chamber is further overlaid with (iii) areas (205) designated by early meet late (EML) LAT range; and
wherein the system further comprises:
a processor (28), which is configured to:
identify a set of shortest paths (212, 213, 214) on the anatomical surface between different EML areas;
select one or more ranges (204) of LAT values that are **characterized by** lowest prevalence over the data points of the EP map;
generate complex tags (218) for the positions having the LAT values within the one or more ranges of LAT values having the lowest prevalence;
select a subset of the shortest paths (212) based on (i) density of the complex tags along the shortest paths and (ii) directions of the activation wave velocity vectors relative to each of the shortest paths; and
present the selected subset of the shortest paths as candidate slow-conduction areas for ablation.

10. The system according to claim 9, wherein the processor is configured to select the one or more ranges of the LAT values by selecting one or more LAT bins of a LAT histogram.

11. The system according to claim 9, wherein the processor is configured to select the subset of the shortest paths by filtering out a path longer than a predefined path length.

12. The system according to claim 9, wherein the interface is configured to receive the EP map with the areas designated by early meet late (EML) LAT range by receiving areas bounded by a curve of blocked conduction (215).

13. The system according to claim 9, wherein the processor is configured to select the shortest paths based on the direction of the activation wave velocity vectors by filtering out a path for which the activation wave velocity vectors are orthogonal to a tangent to the path, up to a given angular tolerance.

14. The system according to claim 9, wherein the processor is configured to generate the complex tags by annotating electrograms at fractioned regions, and extracting LAT values using the annotated electrograms.

15. The system according to claim 9, wherein the cardiac chamber is an atrium exhibiting an atrial flutter or wherein the cardiac chamber is a ventricle exhibiting ventricular tachycardia.

## Patentansprüche

1. Verfahren zum Identifizieren von Kandidatenstellen für eine Ablation, das Verfahren umfassend:
Empfangen einer elektrophysiologischen (EP) Karte (202), umfassend eine anatomische Oberfläche einer Herzkammer, die mit (i) Aktivierungswellengeschwindigkeitsvektoren (207) und (ii) Datenpunkten überlagert ist, umfassend Positionen auf der anatomischen Oberfläche und jeweilige lokale Aktivierungszeiten (LAT) ,
**dadurch gekennzeichnet, dass** die anatomische Oberfläche der Herzkammer ferner mit (iii) Bereichen (205) überlagert ist, die durch den Early-Meet-Late-LAT-Bereich (EML-LAT-Bereich) markiert sind; wobei das Verfahren ferner umfasst:
Identifizieren eines Satzes von kürzesten Pfaden (212, 213, 214) auf der anatomischen Oberfläche zwischen unterschiedlichen EML-Bereichen;
Auswählen eines oder mehrerer Bereiche (204) von LAT-Werten, die **gekennzeichnet sind durch** niedrigste Prävalenz über die Datenpunkte der EP-Karte;
Generieren komplexer Tags (218) für die Positionen, die LAT-Werte innerhalb des einen oder der mehreren Bereiche von LAT-Werten aufweisen, die die niedrigste Prävalenz aufweisen;
Auswählen eines Teilsatzes der kürzesten Pfade (212) basierend auf (i) einer Dichte der komplexen Tags entlang der kürzesten Pfade und (ii) Richtungen der Aktivierungswellengeschwindigkeitsvektoren relativ zu jedem der kürzesten Pfade; und
Darstellen des ausgewählten Teilsatzes der kürzesten Pfade als Kandidaten für langsam leitende Bereiche für die Ablation.

2. Verfahren nach Anspruch 1, wobei das Auswählen des einen oder der mehreren Bereiche der LAT-Werte das Auswählen eines oder mehrerer LAT-Bins eines LAT-Histogramms umfasst.

3. Verfahren nach Anspruch 1, wobei das Auswählen des Teilsatzes der kürzesten Pfade ein Herausfiltern eines Pfads umfasst, der länger als eine vordefinierte Pfadlänge ist.

4. Verfahren nach Anspruch 1, wobei das Empfangen der EP-Karte mit den Bereichen, die durch den Early-Meet-Late-LAT-Bereich (EML-LAT-Bereich) markiert sind, das Empfangen von Bereichen umfasst, die durch eine Kurve einer blockierten Leitung (215) begrenzt sind.

5. Verfahren nach Anspruch 1, wobei das Auswählen der kürzesten Pfade basierend auf der Richtung der Aktivierungswellengeschwindigkeitsvektoren das Herausfiltern eines Pfades umfasst, für den die Aktivierungswellengeschwindigkeitsvektoren bis zu einer gegebenen Winkeltoleranz parallel zu einer Tangente an den Pfad verlaufen.

6. Verfahren nach Anspruch 1, wobei das Generieren der komplexen Tags ein Annotieren von Elektrogrammen in fraktionierten Regionen und ein Extrahieren von LAT-Werten unter Verwendung der annotierten Elektrogramme umfasst.

7. Verfahren nach Anspruch 1, wobei die Herzkammer ein Vorhof ist, der ein Vorhofflattern zeigt.

8. Verfahren nach Anspruch 1, wobei die Herzkammer ein Ventrikel ist, der eine ventrikuläre Tachykardie zeigt.

9. System zum Identifizieren von Kandidatenstellen für die Ablation, das System umfassend:
eine Schnittstelle (35), die konfiguriert ist, um eine elektrophysiologische (EP) Karte zu empfangen, umfassend eine anatomische Oberfläche einer Herzkammer, die mit (i) Aktivierungswellengeschwindigkeitsvektoren (207) und (ii) Datenpunkten überlagert ist, umfassend Positionen auf der anatomischen Oberfläche und jeweilige lokale Aktivierungszeiten (LAT),
**dadurch gekennzeichnet, dass** die anatomische Oberfläche der Herzkammer ferner mit (iii) Bereichen (205) überlagert ist, die durch den Early-Meet-Late-LAT-Bereich (EML-LAT-Bereich) markiert sind; und wobei das System ferner umfasst:
einen Prozessor (28), der konfiguriert ist zum:
Identifizieren eines Satzes von kürzesten Pfaden (212, 213, 214) auf der anatomischen Oberfläche zwischen unterschiedlichen EML-Bereichen;
Auswählen eines oder mehrerer Bereiche (204) von LAT-Werten aus, die **gekennzeichnet sind durch** niedrigste Prävalenz über die Datenpunkte der EP-Karte;
Generieren komplexer Tags (218) für die Positionen, die die LAT-Werte innerhalb des einen oder der mehreren Bereiche von LAT-Werten aufweisen, die die niedrigste Prävalenz aufweisen;
Auswählen eines Teilsatzes der kürzesten Pfade (212) basierend auf (i) der Dichte der komplexen Tags entlang der kürzesten Pfade und (ii) den Richtungen der Aktivierungswellengeschwindigkeitsvektoren relativ zu jedem der kürzesten Pfade; und
Präsentieren des ausgewählten Teilsatzes der kürzesten Pfade als Kandidaten für langsam leitende Bereiche für die Ablation.

10. System nach Anspruch 9, wobei der Prozessor konfiguriert ist, um den einen oder die mehreren Bereiche der LAT-Werte durch Auswählen eines oder mehrerer LAT-Bins eines LAT-Histogramms auszuwählen.

11. System nach Anspruch 9, wobei der Prozessor konfiguriert ist, um den Teilsatz der kürzesten Pfade durch Herausfiltern eines Pfads auszuwählen, der länger als eine vordefinierte Pfadlänge ist.

12. System nach Anspruch 9, wobei die Schnittstelle konfiguriert ist, um die EP-Karte mit den Bereichen zu empfangen, die durch den Early-Meet-Late-LAT-Bereich (EML-LAT-Bereich) durch Empfangen von Bereichen markiert ist, die durch eine Kurve der blockierten Leitung (215) begrenzt sind.

13. System nach Anspruch 9, wobei der Prozessor konfiguriert ist, um die kürzesten Pfade basierend auf der Richtung der Aktivierungswellengeschwindigkeitsvektoren durch Herausfilter eines Pfads auszuwählen, für den die Aktivierungswellengeschwindigkeitsvektoren bis zu einer gegebenen Winkeltoleranz orthogonal zu einer Tangente des Pfads sind.

14. System nach Anspruch 9, wobei der Prozessor konfiguriert ist, um die komplexen Tags durch Annotieren der Elektrogramme in fraktionierten Regionen und Extrahieren der LAT-Werte unter Verwendung der annotierten Elektrogramme zu generieren.

15. System nach Anspruch 9, wobei die Herzkammer ein Vorhof ist, der ein Vorhofflattern zeigt, oder wobei die Herzkammer ein Ventrikel ist, der eine ventrikuläre Tachykardie zeigt.

## Revendications

1. Procédé permettant d'identifier des localisations candidates pour ablation, le procédé comprenant :
la réception d'une carte électrophysiologique (EP) (202) comprenant une surface anatomique d'une chambre cardiaque recouverte avec (i) des vecteurs de vitesse d'onde d'activation (207), et (ii) des points de données comprenant des positions sur la surface anatomique et des temps d'activation locale (LAT) respectifs,
**caractérisé en ce que** la surface anatomique de la chambre cardiaque est en outre recouverte avec (iii) des zones (205) désignées par une plage de LAT de rencontre entre précoce et tardif (EML) ; dans lequel le procédé comprend en outre :
l'identification d'un ensemble de trajets les plus courts (212, 213, 214) sur la surface anatomique entre les différentes zones EML ;
la sélection d'une ou plusieurs plages (204) de valeurs LAT qui sont **caractérisées par** une prévalence la plus basse parmi les points de données de la carte EP ;
la génération d'étiquettes complexes (218) pour les positions ayant les valeurs LAT au sein de la ou des plages de valeurs LAT ayant la prévalence la plus basse ;
la sélection d'un sous-ensemble des trajets les plus courts (212) en fonction de (i) la densité des étiquettes complexes le long des trajets les plus courts et (ii) des directions des vecteurs de vitesse d'onde d'activation par rapport à chacun des trajets les plus courts ; et
la présentation du sous-ensemble sélectionné des trajets les plus courts en tant que zones à conduction lente candidates pour ablation.

2. Procédé selon la revendication 1, dans lequel la sélection de la ou des plages des valeurs LAT comprend la sélection d'un ou plusieurs compartiments LAT d'un histogramme LAT.

3. Procédé selon la revendication 1, dans lequel la sélection du sous-ensemble des trajets les plus courts comprend l'élimination par filtrage d'un trajet plus long qu'une longueur de trajet prédéfinie.

4. Procédé selon la revendication 1, dans lequel la réception de la carte EP avec les zones désignées par une plage LAT de rencontre entre précoce et tardif (EML) comprend la réception de zones délimitées par une courbe de conduction bloquée (215).

5. Procédé selon la revendication 1, dans lequel la sélection des trajets les plus courts en fonction de la direction des vecteurs de vitesse d'onde d'activation comprend l'élimination par filtrage d'un trajet pour lequel les vecteurs de vitesse d'onde d'activation sont parallèles à une tangente au trajet, jusqu'à une tolérance angulaire donnée.

6. Procédé selon la revendication 1, dans lequel la génération des étiquettes complexes comprend l'annotation d'électrogramme au niveau de régions fractionnées, et l'extraction des valeurs LAT à l'aide des électrogrammes annotés.

7. Procédé selon la revendication 1, dans lequel la chambre cardiaque est une oreillette présentant un flutter auriculaire.

8. Procédé selon la revendication 1, dans lequel la chambre cardiaque est un ventricule présentant une tachycardie ventriculaire.

9. Système permettant d'identifier des localisations candidates pour ablation, le système comprenant :
une interface (35) configurée pour recevoir une carte électrophysiologique (EP) comprenant une surface anatomique d'une chambre cardiaque recouverte avec (i) des vecteurs de vitesse d'onde d'activation (207), et (ii) des points de données comprenant des positions sur la surface anatomique et des temps d'activation locale (LAT) respectifs,
**caractérisé en ce que** la surface anatomique de la chambre cardiaque est en outre recouverte avec (iii) des zones (205) désignées par une plage de LAT de rencontre entre précoce et tardif (EML) ; et dans lequel le système comprend en outre :
un processeur (28) qui est configuré pour :
identifier un ensemble de trajets les plus courts (212, 213, 214) sur la surface anatomique entre les différentes zones EML ;
sélectionner une ou plusieurs plages (204) de valeurs LAT qui sont **caractérisées par** une prévalence la plus basse parmi les points de données de la carte EP ;
générer des étiquettes complexes (218) pour les positions ayant les valeurs LAT au sein de la ou des plages de valeurs LAT ayant la prévalence la plus basse ;
sélectionner un sous-ensemble des trajets les plus courts (212) en fonction de (i) la densité des étiquettes complexes le long des trajets les plus courts et (ii) des directions des vecteurs de vitesse d'onde d'activation par rapport à chacun des trajets les plus courts ; et
présenter le sous-ensemble sélectionné des trajets les plus courts en tant que zones à conduction lente candidates pour ablation.

10. Système selon la revendication 9, dans lequel le processeur est configuré pour sélectionner la ou les plages des valeurs LAT en sélectionnant un ou plusieurs compartiments LAT d'un histogramme LAT.

11. Système selon la revendication 9, dans lequel le processeur est configuré pour sélectionner le sous-ensemble des trajets les plus courts en éliminant par filtrage un trajet plus long qu'une longueur de trajet prédéfinie.

12. Système selon la revendication 9, dans lequel l'interface est configurée pour recevoir la carte EP avec les zones désignées par une plage LAT de rencontre entre précoce et tardif (EML) en recevant des zones délimitées par une courbe de conduction bloquée (215).

13. Système selon la revendication 9, dans lequel le processeur est configuré pour sélectionner les trajets les plus courts en fonction de la direction des vecteurs de vitesse d'onde d'activation en éliminant par filtrage un trajet pour lequel les vecteurs de vitesse d'onde d'activation sont orthogonaux à une tangente au trajet, jusqu'à une tolérance angulaire donnée.

14. Système selon la revendication 9, dans lequel le processeur est configuré pour générer les étiquettes complexes par annotation d'électrogrammes au niveau de régions fractionnées, et extraction des valeurs LAT à l'aide des électrogrammes annotés.

15. Système selon la revendication 9, dans lequel la chambre cardiaque est une oreillette présentant un flutter auriculaire ou dans lequel la chambre cardiaque est un ventricule présentant une tachycardie ventriculaire.
